# EUROPÄISCHE PATENTSCHRIFT

(11) **EP 0 653 423 B1**
(45) Veröffentlichungstag und Bekanntmachung des Hinweises auf die Patenterteilung: **16.06.1999**
(21) Anmeldenummer: 94117328.8
(22) Anmeldetag: 03.11.1994
(51) Int. Cl.: C07D 239/96

(54) **Verfahren zur Herstellung von substituierten Chinazolin-2,4-dionen**
Process for the preparation of substituted quinazoline-2,4-diones
Procédé de préparation de quinazolines-2,4-diones substituées

(30) Priorität: 17.11.1993 DE 4339209
(43) Veröffentlichungstag der Anmeldung: 17.05.1995
(73) Patentinhaber: Clariant GmbH, 65929 Frankfurt am Main (DE)
(72) Erfinder: Cosmo, Robert, Dr., D-64291 Darmstadt (DE); Tronich, Wolfgang, Dr., D-65817 Eppstein (DE)

(56) Entgegenhaltungen:
- EP-A- 0 040 793
- EP-A- 0 176 333
- EP-A- 0 183 458
- EP-A- 0 260 817
- EP-A- 0 546 206
- DE-A- 3 712 782
- CHEMICAL ABSTRACTS, vol. 83, no. 11, 15. September 1975, Columbus, Ohio, US; abstract no. 97348u, Seite 596 ;Spalte 2 ; & JP-A-7 514 689 (RES.INSTIT. FOR PRODUCT. DEVELOP.) 15. Februar 1975
- : "", EUR.J.MED.CHEM., Paris, , Band 9, Nr. , Seiten 263 - 268
- : "", J.HETEROCYCL.CHEM., , , Band 19, Nr. , Seiten 269 - 272

## Beschreibung

Substituierte Chinazolin-2,4-dione der allgemeinen Formel (I) sind interessante Zwischenprodukte für Pharmaceutiza und Pflanzenschutzmittel (US PS 4.405.623; GB 1.059.271; EP 360.417).

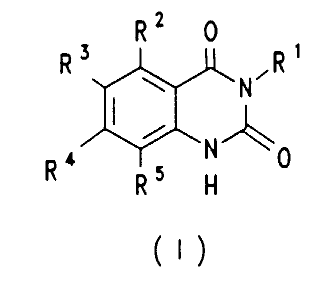

Die Herstellung von (I) erfolgt üblicherweise in zwei Reaktionsschritten: 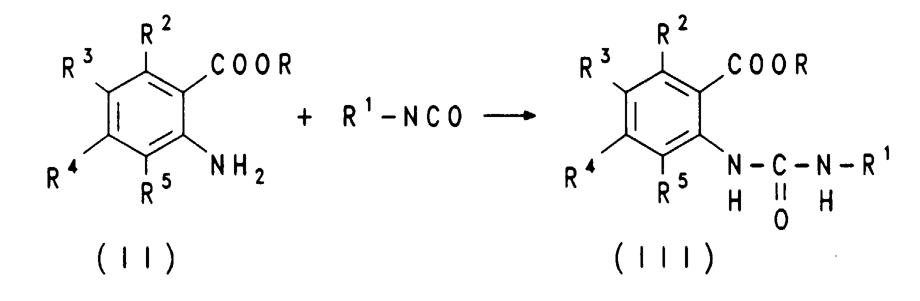 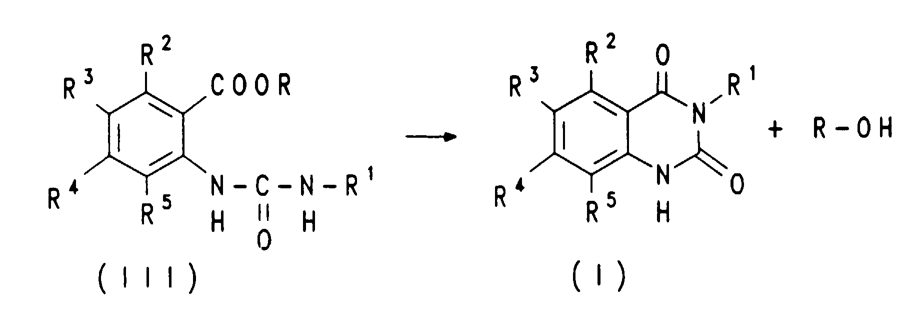 In erster Stufe wird durch Umsetzung einer - gegebenenfalls substituierten - Anthranilsäure oder eines Anthranilsäurealkylesters der allgemeinen Formel (II) mit einem Arylisocyanat in einem, gegenüber Isocyanaten inerten Reaktionsmedium die Zwischenstufe (III) erhalten.

Im Falle von Anthranilsäuren erhält man so N-Arylcarbamoylanthranilsäuren (III, R = H), die zwischenisoliert, gegebenenfalls durch Umkristallisation gereinigt werden und dann in einem zweiten Reaktionsschritt, z. B. in Polyphosphorsäure innerhalb von 5 Stunden bei 150°C (EP 360.417 A1) oder einem protischen, organischen Medium, wie z. B. Ethanol in Gegenwart überschüssiger starker Mineralsäure, bevorzugt gasförmiger Salzsäure, zu Chinazolin-2,4-dionen der allgemeinen Formel (I), ringgeschlossen werden (GB 1.059.271, Beispiel 3).

In entsprechender Weise liefern die Anthranilsäurealkylester bei der Umsetzung mit Arylisocyanaten N-Arylcarbamoylanthranilsäurealkylester (III, R = Alkyl), die zwischenisoliert werden und in analoger Weise wie die Säuren zu den Chinazolindionen cyclisiert werden können. Für Verbindungen (III), R = Alkyl ist es auch bekannt, die Cyclisierung zu (I), in protischen Medien wie z. B. Ethanol oder Methanol in Gegenwart von wäßrigem Natriumhydroxid durchzuführen [DOS 1.804.391 (Beispiel 4); J. Heterocycl. Chem., 19 (2) S. 269 (1982)].

Diese Herstellverfahren für substituierte Chinazolin-2,4-dione haben jedoch den wesentlichen Nachteil, daß die Herstellung der Zwischenstufe (III) stets in einem anderen Reaktionsmedium erfolgt als die Cyclisierung von (III) zu (I).

Dieses Tatsache beruht offensichtlich auf dem Vorurteil, daß der Ringschluß von (III) zu (I) nur in protischen, stark polaren Medien durchgeführt werden kann, wie z. B. in Alkoholen, starken Mineralsäuren oder alkoholisch-wäßrig alkalischen Medien. Diese Medien sind jedoch ihrerseits erwiesenermaßen für die Herstellung von (III) aus (II) und Arylisocyanaten ungeeignet, da sie mit Isocyanaten selbst äußerst heftig reagieren. Dieser Umstand führt somit zu der Verwendung unterschiedlicher Reaktionsmedium für die Herstellung von (III) und (I) und damit zwangsläufig zu erhöhtem technischem und wirtschaftlichem Aufwand, der mit einem Wechsel des Reaktionsmediums und einer Produktzwischenisolierung naturgemäß verbunden ist.

In Europ. J. Med. Chem., 9, S. 263 (1974) wurde beschrieben, daß die Umsetzung von Anthranilsäuremethylester (II, R=CH₃, R², R³, R⁴, R⁵ =H) mit Arylisocyanaten zum N-Arylcarbamoylanthranilsäuremethylester (III, R = CH₃) und die anschließende Cyclisierung zu Chinazolin-2,4-dionen (I) in demselben Reaktionsmedium (Benzol oder Toluol) durchführbar ist. Dabei werden unterstöchiometrische Mengen Triethylamin als Cyclisierungskatalysator eingesetzt. Die Reaktion kann auch ohne Lösungsmittel ablaufen. Dieses Verfahren hat jedoch den Nachteil, daß hohe Reaktionstemperaturen bzw. lange Reaktionszeiten notwendig sind, um das Zwischenprodukt N-Arylcarbamoylanthranilsäurealkylester (III, R = alkyl) in das Chinazolin-2,4-dion (I) zu überführen. Die Produkte dieses Verfahrens sind im allgemeinen mit dem Zwischenprodukt N-Arylcarbamoylanthranilsäurealkylester (III, R = alkyl) verunreinigt. Nur durch Anwendung von überschüssigem Triethylamin kann im allgemeinen eine vollständige Cyclisierung erzielt werden.

Die bereits erwähnte Cyclisierung von (III) mit R = Alkyl; zu (I) in Methanol oder Ethanol als Reaktionsmedium und mit einer überstöchiometrischen Menge an ca. 5 bis 10 %igem wäßrigem Natriumhydroxid [DOS 1.804.391 (Beispiel 4, S. 15); J. Heterocycl. Chem. 19 (2) S. 269 (1982)] hat trotz sehr kurzer Reaktionszeiten und guter Ausbeuten den Nachteil, daß dabei zunächst das Natriumsalz des Chinazolin-2,4-dion erhalten wird, welches anschließend durch Behandlung mit einer Mineralsäure bzw. einer organischen Säure in das freie Dion überführt werden muß, wobei 1 Mol unerwünschtes, abwasserbelastendes Natriumsalz der Mineralsäure bzw. der organischen Säure entsteht.

In der JP-A-75,014,689 werden 1-N-substituierte Chinazolin-2,4-dione hergestellt, indem zunächst der Anthranilsäureester im Lösemittel gelöst und mit einem Alkalimetall oder einer basischen Alkalimetallverbindung versetzt und anschließend mit einem Isocyanat umgesetzt wird. Die erzielbaren Ausbeuten sind jedoch unbefriedigend.

Es bestand somit ein großer Bedarf nach einem Verfahren, das es erlaubt, substituierte Chinazolin-2,4-dione in hoher Ausbeute und Reinheit auf technisch einfache Weise bei geringer Umweltbelastung zu erhalten.

Diese Aufgabe wird gelöst durch ein Verfahren zur Herstellung von Chinazolin-2,4-dionen der allgemeinen Formel (I) 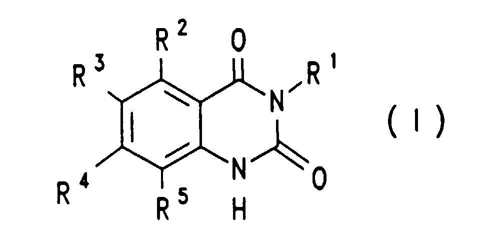 worin R¹ Aryl und R², R³, R⁴, R⁵ unabhängig voneinander Halogen, Alkyl, Alkoxy oder Wasserstoff bedeuten, dadurch gekennzeichnet, daß man Anthranilsäureester der allgemeinen Formel (II) 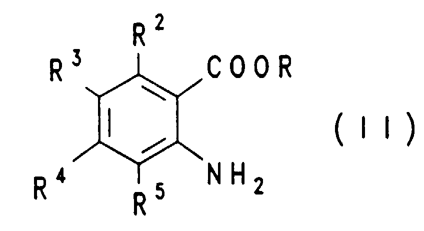 worin R gleich Alkyl bedeutet und R² bis R⁵ die oben angegebene Bedeutung besitzen, in einem aprotischen Reaktionsmedium mit Arylisocyanaten R¹-N = C = O zu N-Arylcarbamoylanthranilsäureestern der allgemeinen Formel (III) 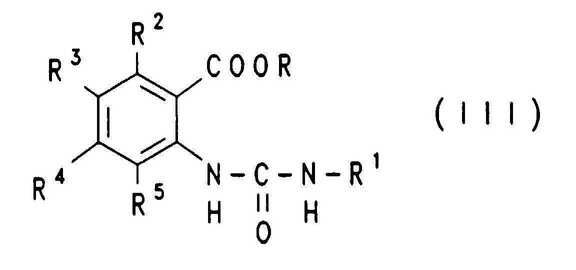 worin R, R¹ bis R⁵ die oben angegebene Bedeutung besitzen, umsetzt und diese in Gegenwart von Alkali- oder Erdalkalimetallakoholaten, -amiden, -hydriden oder Tetraalkylammoniumhydroxiden cyclisiert. Von besonderem Interesse ist das Verfahren zur Herstellung von Verbindungen der Formel I worin R¹ gleich Phenyl ist, das durch Halogen, (C₁-C₄)-Alkyl, (C₁-C₄)-Alkoxy substituiert sein kann, R gleich (C₁-C₃)-Alkyl bedeutet und R² bis R⁵ unabhängig voneinander Wasserstoff, (C₁-C₃)-Alkyl, (C₁-C₃)-Alkoxy, Fluor, Chlor oder Brom bedeuten. Hierbei sind wiederum die Verbindungen wichtig worin R² bis R⁵ unabhängig voneinander Wasserstoff, Fluor, Chlor oder Methyl bedeuten.

Die Cyclisierung von (III) zu (I) verläuft mit hervorragenden Ausbeuten in aprotischen Reaktionsmedien, die als solche inert gegenüber Isocyanaten sind. Bevorzugt werden dieselben Reaktionsmedien verwendet, in welchen auch die Verbindung (III) hergestellt wird, wie z. B. aromatische, aliphatische oder cycloaliphattische Kohlenwasserstoffe, Heterocyclen oder Ketone, insbesondere einkernige Alkylaromaten oder, bei Normalbedingungen flüssige Alkane und Cycloalkane. In vielen Fällen haben sich Toluol und Xylole in reiner Form oder als Isomerengemisch bewährt.

Damit besteht die vorteilhafte Möglichkeit, die Umsetzung von (II) mit dem Arylisocyanat zu (III) und die Cyclisierung von (III) zu (I) in einem und demselben Reaktionsmedium bei niedrigen Temperaturen mit sehr kurzen Reaktionszeiten durchzuführen; hierbei kann auf eine Zwischenisolierung von (III) verzichtet werden. Jedoch ist es auch möglich (III), z. B. aus Gründen einer zusätzlichen Reinigung, zu isolieren und anschließend erneut in frischem Reaktionsmedium zu (I) zu cyclisieren.

Als Anthranilsäureester werden im allgemeinen die Alkylester eingesetzt; bevorzugt kommen substituierte Anthranilsäuremethylester, insbesondere fluorsubstituierte Anthranilsäuremethylester, deren Herstellung beispielsweise in der deutschen Patentanmeldung P 43 34 432.1 beschrieben ist, zum Einsatz.

Die als Base verwendeten Alkali- oder Erdalkalimetallalkoholate können in Substanz oder als Lösungen in den entsprechenden Alkoholen eingesetzt werden. In vielen Fällen hat sich der Einsatz von Natriummethylat in Methanol bewährt.

Verwendet man Alkali- oder Erdalkalimetallhydride oder Amide, so können diese ebenfalls in Substanz oder als Suspension im aprotischen Solvent eingesetzt werden.

Die verwendeten Basen werden üblicherweise in Mengen von 1 bis 95 Mol-% bezogen auf die N-Arylcarbamoylanthranilsäureester (III) eingesetzt. Es hat sich als günstig erwiesen, Mengen von 1 bis 20 Mol-%, insbesondere 5 bis 10 Mol-% zu verwenden.

Gegenüber dem in Europ. J. Med. Chem., 9, S. 263 (1974) beschriebenen Herstellweg hat das erfindungsgemäße Verfahren zur Herstellung von Chinazolin-2,4-dionen folgende wesentliche Vorteile: Beim Einsatz der erfindungsgemäßen Basen sind deutlich kürzere Reaktionszeiten und niedrigere Temperaturen zur vollständigen Cyclisierung des N-Arylcarbamoylanthranilsäurealkylesters (III) zum Chinazolin-2,4-dion (I) erforderlich als beim Einsatz von Triethylamin (siehe Beispiel 1 und 5.1, sowie Beispiele 5.1 und 6.2). Außerdem werden die Chinazolin-2,4-dione der Formel (I) in besserer Ausbeute und in besserer Reinheit erhalten.

Die Reaktionstemperatur für die Umsetzung des Anthranilsäureethylester mit dem Arylisocyanat liegt im allgemeinen zwischen ca. 20°C und dem Siedepunkt des verwendeten Reaktionsmediums, bevorzugt in einem Bereich zwischen 30 und 120°C. Die Reaktionstemperatur der Cyclisierung (III) zu (I) wird zweckmäßig so gewählt, daß der freiwerdende Alkohol aus dem Reaktionsmedium abdestilliert; im Falle des bevorzugten Methyl- oder Ethylesters von (II) entspricht dies einer Reaktionstemperatur oberhalb des Siedepunktes von Methanol bzw. Ethanol.

### Beispiele

### Beispiel 1: Herstellung von 3-(4-Chlorphenyl)-2,4(1H,3H)-chinazolindion

In einer Lösung von Anthranilsäuremethylester (15,1 g, 0,10 mol) in Toluol (100 ml) wird eine Lösung von p-Chlorphenylisocyanat (15,3 g, 0,10 mol) in Toluol (50 ml) bei Raumtemperatur zudosiert. Nach beendeter Zugabe wird der Ansatz 2 Stunden bei 90°C gerührt. Es werden 1,80 ml einer 30 %igen Lösung von methanolischem Natriumethylat (0,010 mol) zudosiert und weitere 1 Stunde bei 90°C gerührt, wobei Methanol abdestilliert wird. Nach Abkühlung wird das Produkt abgesaugt und mit 3 x 20 ml Toluol gewaschen. Der Toluol-feuchte Niederschlag wird mit verdünnter Schwefelsäure versetzt und der Rest-Xylol durch azeotrope Destillation entfernt. Das Produkt wird anschließend abgesaugt, mit Wasser gewaschen und getrocknet. Es werden 26,8 g 3-(4-Chlorphenyl)-2,4(1H,3H)-chinazolindon (Ausbeute: 98,3 % d. Th.) mit Schmelzpunkt 290 bis 292°C erhalten.

### Beispiel 2: Herstellung von 3-(4-Tolyl)-2,4(1H,3H)-chinazolindion

In einer Lösung von Anthranilsäureethylester (165,2 g, 1,00 mol) in Xylol (600 ml) wird eine Lösung von p-Tolylisocyanat (133,2 g, 1,00 mol) in Xylol (300 ml) bei Raumtemperatur zudosiert. Nach beendeter Zugabe wird der Ansatz 2,5 Stunden bei 90°C gerührt. Es werden 18,0 ml einer 30 %igen Lösung von methanolischem Natriummethylat (0,10 mol) zudosiert und weitere 2 Stunden bei 90°C gerührt, wobei Ethanol bzw. Methanol abdestilliert wird. Nach Abkühlung wird das Produkt abgesaugt und mit 3 x 75 ml Xylol gewaschen. Der Xylol-feuchte Niederschlag wird mit verdünnter Salzsäure versetzt und der Rest-Xylol durch azeotrope Destillation entfernt. Das Produkt wird anschließend abgesaugt, mit Wasser gewaschen und getrocknet. Es werden 233,0 g 3-(4-Tolyl)-2,4(1H,3H)-chinazolindion (Ausbeute: 92,4 % d. Th.) mit Schmelzpunkt 267°C (lit 265 bis 266°C) erhalten.

### Beispiel 3: Herstellung von 6-Fluor-3-(3-Tolyl)-2,4(1H,3H)-chinazolindion

In einer Lösung von 4-Fluoranthranilsäureethylester (45,8 g, 0,25 mol) in Xylol (250 ml) wird eine Lösung von m-Tolylisocyanat (33,3 g, 0,25 mol) in Xylol (100 ml) bei Raumtemperatur zudosiert. Nach beendeter Zugabe wird der Ansatz 2,5 Stunden bei 90°C gerührt. Es werden 32,5 ml einer 20 %igen Lösung von wäßrigem Tetrabutylammoniumhydroxid (0,025 mol) zudosiert und weitere 2 Stunden bei 90°C gerührt, wobei Ethanol bzw. Methanol abdestilliert wird. Nach Abkühlung wird das Produkt abgesaugt und mit 3 x 30 ml Xylol gewaschen. Der Xylol-feuchte Niederschlag wird mit verdünnter Salzsäure versetzt und der Rest-Xylol durch azeotrope Destillation entfernt. Das Produkt wird anschließend abgesaugt, mit Wasser gewaschen und getrocknet. Es werden 62,0 g 6-Fluor-3-(3-tolyl)-2,4(1H,3H)-chinazolindion (Ausbeute: 91,7 % d. Th.) mit Schmelzpunkt 278 bis 279°C erhalten.

### Beispiel 4: Herstellung von 3-(2-Tolyl)-6,7-dimethoxy-2,4(1H,3H)-chinazolidion

In einer Lösung von 4,5-Dimethoxyanthranilsäuremethylester (21,1 g, 0,10 mol) in Toluol (70 ml) wird eine Lösung von o-Tolylisocyanat (13,3 g, 0,10 mol) in Toluol (40 ml) bei Raumtemperatur zudosiert. Nach beendeter Zugabe wird der Ansatz 2 Stunden bei 90°C gerührt. Es werden 1,80 ml einer 30 %igen Lösung von methanolischem Natriumethylat (0,010 mol) zudosiert und weitere 1 Stunde bei 90°C gerührt, wobei Methanol abdestilliert wird. Nach Abkühlung wird das Produkt abgesaugt und mit 3 x 30 ml Toluol gewaschen. Der Toluol-feuchte Niederschlag wird mit verdünnter Schwefelsäure versetzt und der Rest-Xylol durch azeotrope Destillation entfernt. Das Produkt wird anschließend abgesaugt, mit Wasser gewaschen und getrocknet. Es werden 29,0 g 3-(2-Tolyl)-6,7-dimethoxy-2,4(1H,3H)-chinazolindon (Ausbeute: 92,9 % d. Th.) mit Schmelzpunkt 282 bis 284°C erhalten.

### Beispiel 5.1 - 5.4: Herstellung von 3-(2,4-Dichlorphenyl)-6-fluor-2,4(1H,3H)-chinazolindion in verschiedenen Lösungsmitteln

### Beispiel 5.1: Herstellung von 3-(2,4-Dichlorphenyl)-6-fluor-2,4(1H,3H)-chinazolindion in Xylol

In einer Lösung von 5-Fluoranthranilsäureethylester (45,2 g, 0,25 mol) in Xylol (210 ml) wird eine Lösung von 2,4-Diclorphenylisocyanat (47,0 g, 0,25 mol) in Xylol (130 ml) bei Raumtemperatur zudosiert. Nach beendeter Zugabe wird der Ansatz 2,5 Stunden bei 90°C gerührt. Es werden 4,50 ml einer 30 %igen Lösung von methanolischem Natriummethylat (0,025 mol) zudosiert und weitere 2 Stunden bei 90°C gerührt, wobei Ethanol bzw. Methanol abdestilliert wird. Nach Abkühlung wird das Produkt abgesaugt und mit 3 x 30 ml Xylol gewaschen. Der Xylol-feuchte Niederschlag wird mit verdünnter Salzsäure versetzt und der Rest-Xylol durch azeotrope Destillation entfernt. Das Produkt wird anschließend abgesaugt, mit Wasser gewaschen und getrocknet. Es werden 73,4 g 3-(2,4-Dichlorphenyl)-6-fluor-2,4(1H,3H)-chinazolindion (Ausbeute: 90,3 % d. Th.) mit Schmelzpunkt 313 bis 314°C erhalten.

### Beispiel 5.2: Herstellung von 3-(2,4-Dichlorphenyl)-6-fluor-2,4(1H,3H)-chinazolindion in Aceton

In einer Lösung von 5-Fluoranthranilsäureethylester (18,3 g, 0,10 mol) in Aceton (70 ml) wird eine Lösung von 2,4-Dichlorphenylisocyanat (18,8 g, 0,10 mol) in Aceton (50 ml) bei Raumtemperatur zudosiert. Nach beendeter Zugabe wird der Ansatz 2,5 Stunden bei 90°C gerührt. Es werden 1,80 ml einer 30 %igen Lösung von methanolischem Natriumethylat (0,010 mol) zudosiert und weitere 2 Stunden unter Rückfluß gerührt. Nach Abkühlung wird das Produkt abgesaugt und mit 3 x 15 ml Aceton gewaschen. Der Xylol-feuchte Niederschlag wird mit verdünnter Salzsäure versetzt und der Rest-Xylol durch azeotrope Destillation entfernt. Das Produkt wird anschließend abgesaugt, mit Wasser gewaschen und getrocknet. Es werden 31,7 g 3-(2,4-Dichlorphenyl)-6-fluor-2,4(1H,3H)-chinazolindion (Ausbeute: 97,5 % d. Th.) mit Schmelzpunkt 313 bis 314°C erhalten.

### Beispiel 5.3: Herstellung von 3-(2,4-Dichlorphenyl)-6-fluor-2,4(1H,3H)-chinazolindion in Hexan

In einer Lösung von 5-Fluoranthranilsäureethylester (18,3 g, 0,10 mol) in Hexan (120 ml) wird 2,4-Dichlorphenylisocyanat (18,8 g, 0,10 mol) portionsweise bei Raumtemperatur eingetragen. Nach beendeter Zugabe wird der Ansatz 2 Stunden unter Rückfluß gerührt. Es werden 3,60 ml einer 30 %igen Lösung von methanolischem Natriummethylat (0,020 mol) zudosiert und weitere 2 Stunden unter Rückfluß gerührt, wobei Ethanol bzw. Methanol abdestilliert wird. Nach Abkühlung wird das Produkt abgesaugt und mit 3 x 15 ml Hexan gewaschen. Der Xylol-feuchte Niederschlag wird mit verdünnter Salzsäure versetzt und der Rest-Hexan durch azeotrope Destillation entfernt. Das Produkt wird anschließend abgesaugt, mit Wasser gewaschen und getrocknet. Es werden 31,2 g 3-(2,4-Dichlorphenyl)-6-fluor-2,4(1H,3H)-chinazolindion (Ausbeute: 96,0 % d. Th.) mit Schmelzpunkt 312 bis 314°C erhalten.

### Beispiel 5.4: Herstellung von 3-(2,4-Dichlorphenyl)-6-fluor-2,4(1H,3H)-chinazolindion in N-Methylpyrrolidon

In einer Lösung von 5-Fluoranthranilsäureethylester (18,3 g, 0,10 mol) in N-Methylpyrrolidon (70 ml) wird eine Lösung von 2,4-Dichlorphenylisocyanat (18,8 g, 0,10 mol) in N-Methylpyrrolidon (50 ml) bei Raumtemperatur zudosiert. Nach beendeter Zugabe wird der Ansatz 2 Stunden bei 90°C gerührt. Es werden 1,80 ml einer 30 %igen Lösung von methanolischem Natriumethylat (0,010 mol) zudosiert und weitere 2 Stunden unter Rückfluß gerührt, wobei Ethanol bzw. Methanol abdestilliert wird. Nach Abkühlung wird das Produkt durch Zugabe von verdünnter Salzsäure (100 ml) ausgefällt. Das Produkt wird anschließend abgesaugt, mit Wasser gewaschen und getrocknet. Es werden 29,8 g 3-(2,4-Dichlorphenyl)-6-fluor-2,4(1H,3H)-chinazolindion (Ausbeute: 91,7 % d. Th.) mit Schmelzpunkt 311 bis 313°C erhalten.

### Beispiel 6.1: Herstellung von 3-(4-Chlorphenyl)-2,4(1H,3H)-chinazolindion in Xylol (Katalysator: Triethylamin) - Analog zum Beispiel 1

In einer Lösung von Anthranilsäureethylester (14,1 g, 0,10 mol) in Toluol (100 ml) wird eine Lösung von p-Chlorphenylisocyanat (15,3 g, 0,10 mol) in Toluol (50 ml) bei Raumtemperatur zudosiert. Nach beendeter Zugabe wird der Ansatz 2 Stunden bei 90°C gerührt. Triethylamin (1,4 ml, 1,0 g, 0,010 mol) wird zudosiert und weitere 3 Stunden bei 90°C gerührt. Nach Abkühlung wird das Produkt abgesaugt und mit 3 x 20 ml Toluol gewaschen und getrocknet. Es werden 21,0 g 3-(4-Chlorphenyl)-2,4(1H,3H)-chinazolindion (Ausbeute: 77,0 % d. Th.) mit Schmelzpunkt 288 bis 290°C erhalten. Nach Dünnschichtchromatographie ist das Produkt mit der Zwischenstufe N-(4-Chlorphenylcarbamoyl)anthranilsäureethylester verunreinigt.

### Beispiel 6.2: Versuch zur Herstellung von 3-(2,4-Dichlorphenyl)-6-fluor-2,4(1H,3H)-chinazolindion in Xylol (Vergleichsbeispiel Katalysator: Triethylamin) - Analog zum Beispiel 5.1

In einer Lösung von 5-Fluoranthranilsäureethylester (45,2 g, 0,25 mol) in Xylol (210 ml) wird eine Lösung von 2,4-Dichlorphenylisocyanat (47,0 g, 0,25 mol) in Xylol (130 ml) bei Raumtemperatur zudosiert. Nach beendeter Zugabe wird der Ansatz 2 Stunden bei 90°C gerührt. Triethylamin (3,5 ml, 2,55 g, 0,025 mol) wird zudosiert. Es werden weitere 2 Stunden bei 90°C gerührt. Nach Abkühlung wird das Produkt abgesaugt und mit 3 x 30 ml Xylol gewaschen und getrocknet. Es werden 86,9 g N-(2,4-Dichlorphenylcarbamoyl)-5-fluoranthranilsäureethylester mit Schmelzpunkt 219 bis 223°C (Zersetzung) erhalten. Das Cyclisierungsprodukt 3-(2,4-Dichlorphenyl)-6-fluor-2,4(1H,3H)-chinazolindion ist durch Dünnschichtchromatographie nicht nachweisbar.

## Patentansprüche

1. Verfahren zur Herstellung von Chinazolin-2,4-dionen der allgemeinen Formel (I) 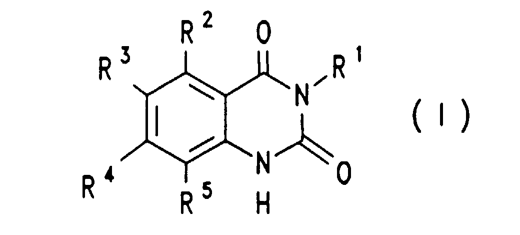 worin R¹ Aryl und R², R³, R⁴, R⁵ unabhängig voneinander Halogen, Alkyl, Alkoxy oder Wasserstoff bedeuten, dadurch gekennzeichnet, daß man Anthranilsäureester der allgemeinen Formel (II) 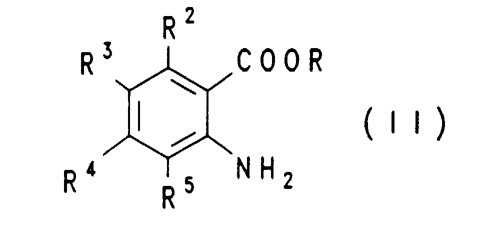 worin R gleich Alkyl bedeutet und R² bis R⁵ die oben angegebene Bedeutung besitzen, in einem aprotischen Reaktionsmedium mit Arylisocyanaten R¹-N = C = O zu N-Arylcarbamoylanthranilsäureestern der allgemeinen Formel (III) 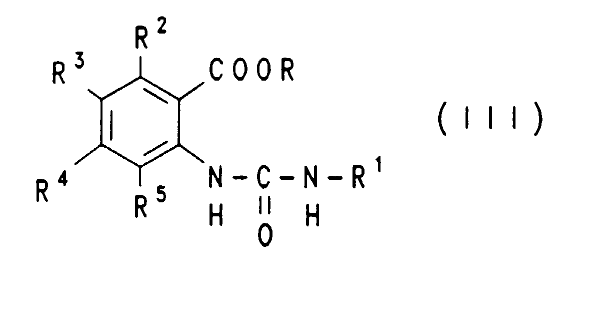 worin R, R¹ bis R⁵ die oben angegebene Bedeutung besitzen, umsetzt und diese in Gegenwart von Alkali- oder Erdalkalimetallakoholaten, -amiden, -hydriden oder Tetraalkylammoniumhydroxiden cyclisiert.

2. Verfahren nach Anspruch 1, dadurch gekennzeichnet, daß R¹ gleich Phenyl, das durch Halogen, (C₁-C₄)-Alkyl, (C₁-C₄)-Alkoxy, substituiert sein kann, R gleich (C₁-C₃)-Alkyl ist und R² bis R⁵ unabhängig voneinander Wasserstoff, (C₁-C₃)-Alkyl, (C₁-C₃)-Alkoxy, Fluor, Chlor oder Brom, insbesondere Wasserstoff, Fluor, Chlor oder Methyl bedeuten.

3. Verfahren nach Anspruch 1 oder 2, dadurch gekennzeichnet, daß man als aprotisches Reaktionsmedium aromatische, cycloaliphatische oder aliphatische Kohlenwasserstoffe, Heterocyclen oder Ketone einsetzt.

4. Verfahren nach Anspruch 1 oder 2, dadurch gekennzeichnet, daß man als aprotisches Reaktionsmedium einen aromatischen Kohlenwasserstoff, insbesondere ein Alkylbenzol, bevorzugt Xylol oder deren Isomerengemisch verwendet.

5. Verfahren nach mindestens einem der Ansprüche 1 bis 4, dadurch gekennzeichnet, daß man die Alkali- oder Erdalkalimetallalkoholate, -amide, -hydride oder Tetraalkylammoniumhydroxide in Mengen von 1 bis 95 Mol-%, insbesondere 1 bis 20 Mol-%, bevorzugt 5 bis 10 Mol-% bezogen auf (III) einsetzt.

6. Verfahren nach mindestens einem der Ansprüche 1 bis 5, dadurch gekennzeichnet, daß Natriummethylat in Methanol eingesetzt wird.

7. Verfahren nach mindestens einem der Ansprüche 1 bis 6, dadurch gekennzeichnet, daß die Umsetzung des Arylisocyanats mit dem Anthranilsäureester bei Temperaturen von 20 bis 150°C, insbesondere 30 bis 120°C durchgeführt wird.

8. Verfahren nach mindestens einem der Ansprüche 1 bis 7, dadurch gekennzeichnet, daß die Reaktionstemperatur der Cyclisierung so gewählt wird, daß der entstehende Alkohol ROH aus dem Reaktionmedium abdestilliert.

## Claims

1. A process for the preparation of quinazoline-2,4-diones of the formula (I) 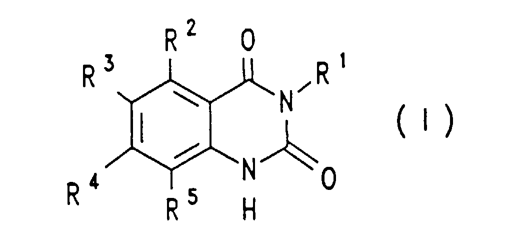 in which R¹ is aryl and R², R³, R⁴ and R⁵ independently of one another are halogen, alkyl, alkoxy or hydrogen, which comprises reacting anthranilic acid esters of the formula (II) 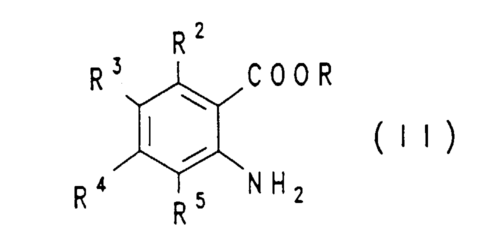 in which R is alkyl and R² to R⁵ have the meaning indicated above, in an aprotic reaction medium with aryl isocyanates R¹-N=C=O to give N-arylcarbamoylanthranilic acid esters of the formula (III) 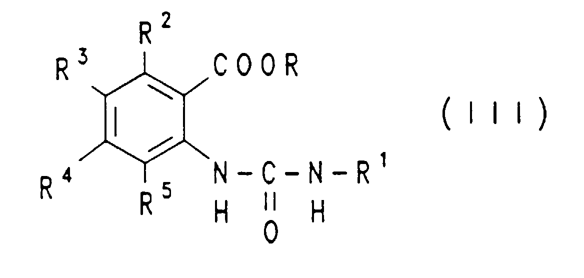 in which R and R¹ to R⁵ have the meaning indicated above, and cyclizing these in the presence of alkali metal or alkaline earth metal alkoxides, amides, hydrides or tetraalkylammonium hydroxides.

2. The process as claimed in claim 1, wherein R¹ is phenyl which can be substituted by halogen, (C₁-C₄) - alkyl or (C₁-C₄)-alkoxy, R is (C₁-C₃)-alkyl and R² to R⁵ independently of one another are hydrogen, (C₁-C₃) -alkyl, (C₁-C₃) -alkoxy, fluorine, chlorine or bromine, in particular hydrogen, fluorine, chlorine or methyl.

3. The process as claimed in claim 1 or 2, wherein the aprotic reaction medium employed is an aromatic, cycloaliphatic or aliphatic hydrocarbon, heterocycle or ketone.

4. The process as claimed in claim 1 or 2, wherein the aprotic reaction medium used is an aromatic hydrocarbon, in particular an alkylbenzene, preferably xylene or its isomer mixture.

5. The process as claimed in at least one of claims 1 to 4, wherein the alkali metal or alkaline earth metal alkoxides, amides, hydrides or tetraalkylammonium hydroxides are employed in amounts from 1 to 95 mol %, in particular 1 to 20 mol %, preferably 5 to 10 mol % based on (III).

6. The process as claimed in at least one of claims 1 to 5, wherein sodium methoxide in methanol is employed.

7. The process as claimed in at least one of claims 1 to 6, wherein the reaction of the aryl isocyanate with the anthranilic acid ester is carried out at temperatures from 20 to 150°C, in particular 30 to 120°C.

8. The process as claimed in at least one of claims 1 to 7, wherein the reaction temperature of the cyclization is chosen such that the resulting alcohol ROH distils off from the reaction medium.

## Revendications

1. Procédé de préparation de quinazolin-2,4-diones de formule générale (I) 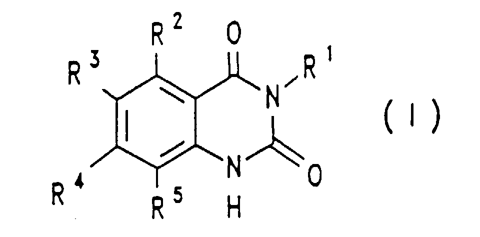 dans laquelle R¹ représente un groupe aryle et R², R³, R⁴, R⁵ représentent indépendamment les uns des autres un atome d'halogène, un groupe alkyle, alcoxy ou un atome d'hydrogène, caractérisé en ce qu'on met à réagir un anthranilate de formule générale (II) 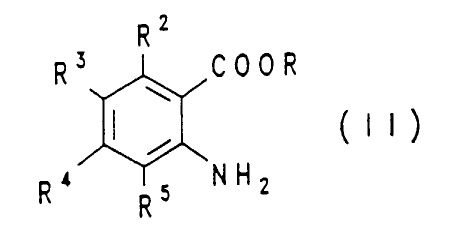 dans laquelle R représente un groupe alkyle et R² à R⁵ possèdent la signification indiquée ci-dessus, dans un milieu réactionnel aprotique avec des isocyanates d'aryle R¹-N=C=O pour donner des N-arylcarbamoylanthranilates de formule générale (III) 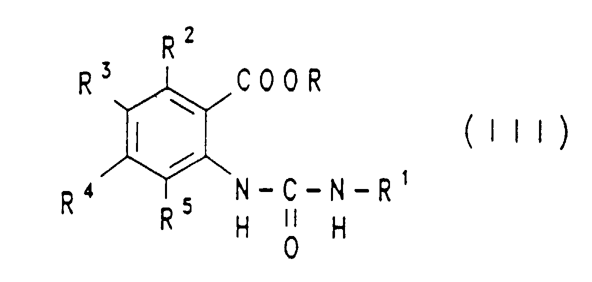 dans laquelle R, R¹ à R⁵ possèdent la signification indiquée ci-dessus, et on cyclise ceux-ci en présence d'alcoolates, d'amides, d'hydrures de métaux alcalins ou alcalino-terreux ou d'hydroxydes de tétraalkylammonium.

2. Procédé selon la revendication 1, caractérisé en ce que R¹ est égal à un groupe phényle, qui peut être substitué par un atome d'halogène, un groupe alkyle en C₁-C₄, alcoxy en C₁-C₄, R représente un groupe alkyle en C₁-C₃ et R² à R⁵ représentent indépendamment les uns des autres un atome d'hydrogène, un groupe alkyle en C₁-C₃, alcoxy en C₁-C₃, un atome de fluor, de chlore ou de brome, en particulier un atome d'hydrogène, de fluor, de chlore ou un groupe méthyle.

3. Procédé selon la revendication 1 ou 2, caractérisé en ce qu'on utilise comme milieu réactionnel aprotique des hydrocarbures aromatiques, cycloaliphatiques ou aliphatiques, des hétérocycles ou des cétones.

4. Procédé selon la revendication 1 ou 2, caractérisé en ce qu'on utilise comme milieu réactionnel aprotique un hydrocarbure aromatique, en particulier un alkylbenzène, de préférence le xylène ou un mélange d'isomères de ceux-ci.

5. Procédé selon au moins l'une des revendications 1 à 4, caractérisé en ce qu'on utilise les alcoolates, amides, hydrures de métaux alcalins ou alcalino-terreux ou les hydroxydes de tétraalkylammonium en quantités de 1 à 95% en mole, en particulier de 1 à 20% en mole, de préférence de 5 à 10% en mole par rapport à (III).

6. Procédé selon au moins l'une des revendications 1 à 5, caractérisé en ce qu'on utilise le méthylate de sodium dans le méthanol.

7. Procédé selon au moins l'une des revendications 1 à 6, caractérisé en ce qu'on réalise la réaction de l'isocyanate d'aryle avec l'anthranilate à des températures de 20 à 150°C, en particulier de 30 à 120°C.

8. Procédé selon au moins l'une des revendications 1 à 7, caractérisé en ce qu'on choisit la température de réaction de façon à éliminer l'alcool ROH formé par distillation du milieu réactionnel.
